Europäisches Patentamt

**European Patent Office**

Office européen des brevets

(19)

(11) Publication number: **0 186 495 B1**

# EUROPEAN PATENT SPECIFICATION

(12)

(45) Date of publication of patent specification: 02.10.91    (51) Int. Cl.⁵: **C12P 19/04**

(21) Application number: 85309417.5

(22) Date of filing: 23.12.85

(54) Production of microbial cellulose.

(30) Priority: 21.12.84 US 684844

(43) Date of publication of application:
02.07.86 Bulletin 86/27

(45) Publication of the grant of the patent:
02.10.91 Bulletin 91/40

(84) Designated Contracting States:
AT BE CH DE FR IT LI LU NL SE

(56) References cited:
US-A- 4 320 198
US-A- 4 378 431

CHEMICAL ABSTRACTS, vol. 89, no. 21, 20th
November 1978, page 457, abstract no.
178103m, Columbus, Ohio, US

CHEMICAL ABSTRACTS, vol. 99, no. 18, 31st
October 1983, page 21, abstract no. 140710a,
Columbus, Ohio, US; P. ZUGENMAIER et al.:
"The structure of cellulose tricarbanilate:
conformation and liquid crystalline behav-
ior"

POLYMER JOURNAL, vol. 7, no. 2, 1975,
pages 137-146, Tokyo, JP; M. TAKAI et al.:
"Biosynthesis of cellulose by Acetobacter
Xylinum. I. Characterizations of bacterial cel-
lulose"

(73) Proprietor: **BOARD OF REGENTS THE UNIVER-
SITY OF TEXAS SYSTEM
Office of General Council, 201 West 7th
Street
Austin, Texas 78701(US)**

(72) Inventor: **Roberts, Eric M.
1409A West 40th Street
Austin Texas 78756(US)**
Inventor: **Hardison, Linda K.
4418 Marathon Street
Austin Texas 78756(US)**
Inventor: **Brown, R. Malcolm
3509 Bonnie
Austin Texas 78703(US)**

(74) Representative: **Clifford, Frederick Alan et al
MARKS & CLERK 57/60 Lincoln's Inn Fields
London WC2A 3LS(GB)**

## Description

This invention relates generally to the production of microbial cellulose. Heretofore, standing cultures of cellulose-producing microorganisms have been used to produce cellulose at the liquid-air interface of such cultures. While the biosynthetic mechanism of microbial cellulose synthesis is incompletely understood, it does appear to have a high demand for oxygen. A standing culture of such microorganisms in a gas-impermeable vessel produces a planar cellulosic film or pellicle in the liquid near where that liquid abuts the air.

The structure of microbial cellulose membranes has been studied by Purz et al (Faserforschung und Textiltechnik 28(4) pp. 155-163, 1977 and 27(11) pp. 561-570, 1976) and determined to be an interwoven and disordered mesh of fibrillar strands with diameters of 50 nm to 100 nm.

Acetobacter xylinum pellicle has apparently been utilized to produce edible gelatinous cubes under the name Nata De Coco by Orientex Merchandising of the Philippines.

According to Bergey's Manual of Systematic Bacteriology (Vol. 1, ed. N.R. Krieg pp. 268-274, Williams and Wilkins, Baltimore, Md. 1984) cellulose synthesizing Acetobacter strains which were formerly classified as Acetobacter aceti, subspecies xylinum are now classified as subspecies of Acetobacter pasteurianus and Acetobacter hansenii. The Bergey reference cited above also refers to studies indicating the static culture favours cellulose synthesizing cells while shake cultures favor cellulose-free mutant cells.

The present invention comprises a process for microbial cellulose production. Both a liquid culture comprising a cellulose-producing microorganism and a structure having a first side and a second side and comprising material permeable to gases but not allowing the passage of microorganisms are provided. The first side of the structure is contacted with an oxygen-containing gas. The second side of the structure is contacted with the liquid culture so that microbial cellulose is produced thereon. The structure may be a shaped mold or cast or may supply oxygen-containing gas to a continually nutrient supplemented culture.

Cellulose-producing microorganisms useful in the the present invention include members of the Acetobacter, Rhizobium, Agrobacterium and Pseudomonas genera (Brown et al. J. Applied Polymer Science: Appl. Polymer Symp. (1983) 37 33-78). The growth of such cellulose-producing micro-organisms with microbial cellulose productions occurs generally in a suitable nutrient medium having a pH between about 3. 5 and about 5.5 when incubated under aerobic conditions and at temperatures between about 20°C and about 30°C. A use of Acetobacter xylinum to coat synthetic fibers with microbial cellulose is disclosed in U.S. 4,378,431, issued to Brown. Many varieties of cellulose-producing microorganisms, particularly Acetobacter xylinum, exist and are virtually ubiquitous in natural surroundings such as damp forests for example.

A suitable nutrient medium for Acetobacter culture is Schramm & Hestrin medium (Schramm et al., J. General Biology 11, pp. 123-129, 1954) comprising about 20 g/1 glucose; 5 g/1 peptone; 5 g/1 yeast extract; 2.7 g/1 anhydrous dibasic sodium phosphate; and 1.15 g/1 citric acid monohydrate. The pH of the medium is adjusted to between about pH 3.5 and about pH 5.5 by the addition of hydrochloric acid. Another suitable nutrient medium comprises about 8 volume percent vinegar, 5 volume percent ethanol and 4 weight percent malt extract. Any of a wide variety of nutrient media having a pH between about 3.5 and about 5.5 are suitable for the practice of the present invention (see Bergey, cited above). Such suitable nutrient media may preferably include a hexose, most preferably glucose, acetic acid and yeast extract. Yet another suitable nutrient medium, adjusted to the above described pH range, is corn steep liquor.

In one embodiment of the present invention, a cellulose-producing microorganism, most preferably Acetobacter xylinum, is inoculated into a volume of suitable nutrient medium. The inoculated nutrient medium is introduced into a shaped mold which is permeable to gases and yet substantially retains the nutrient medium and microorganism to produce a filled mold. The nutrient medium and microorganism may also be separately introduced to the mold and mixed therein. The shaped mold preferably comprises a membrane or film of natural or synthetic polymer, sufficiently porous to allow gaseous diffusion therethrough while substantially retaining the liquid nutrient medium and microorganisms therein. Among the porous materials preferably utilized in the practice of the present invention are polyvinyl chloride, cellulose, cellulose derivatives such as cellulose acetate or cellulose nitrate and polyethylene. Many other materials comprising synthetic or natural polymers well known to those skilled in the art are usable as porous materials to produce the shaped molds, for example, of the present invention.

After the suitable nutrient medium and cellulose producing microorganism have been introduced into the shaped mold, the resultant filled shaped mold is preferably suspended in the air or in an oxygen-enriched gaseous environment or may rest upon a porous surface therein. To minimize evaporative liquid losses, the filled mold is most preferably incubated in an enclosed chamber containing a separate reservoir of water to maintain a humid atmosphere.

As the inoculated filled mold is incubated at a temperature between about 20° C and about 30° C for a period between about 2 days and about 21 days, microbial cellulose is produced and passes from the microorganisms, which themselves are growing and multiplying. The produced microbial cellulose, after sufficient period, forms a relatively uniform shaped object of hydrated microbial cellulose having the form of the mold. The shaped object may then be collected from the mold for use or further processing.

Such shaped objects of hydrated microbial cellulose, after washing and any desired trimming, are usable as, for example, flexible contact lenses, wound dressings or burn dressings or gloves. Examples of further processing of the shaped objects to produce different products include impregnation with proteins, peptides, amino acids, vitamins, other nutrient flavoring agents or texturing agents individually or in combination. Such impregnated shaped microbial cellulose objects function as edible artificial foods or nutritional supplements useful as produced or dried for stabilization and later consumption.

Yet other forms of further processing include, for example, drying the shaped objects to produce particular containers, structural supports, packaging materials or absorbents. In particular cases, the microbial cellulose objects could be subjected to chemical treatment such as chemical derivatization with, for example, acetyl chloride to form cellulose acetate objects which in turn may be physically modified to form a nonwoven cellulosic textile.

In view of the above described aerobic suspension of permeable molds containing nutrient medium inoculated with cellulose-producing microorganisms, other embodiments of the invention are apparent. In a second embodiment of the present invention, a cast, which comprises material permeable to gases and encloses a quantity of oxygen-containing gas, is immersed in nutrient medium inoculated with a cellulose-producing microorganism. The microorganism produces cellulose at the liquid-material interface to substantially coat the cast. After this coating has proceeded to a desired degree, the cast, coated with hydrous microbial cellulose, may be removed and used or processed further, for example by drying, chemical derivatization or cast removal, if desired.

Further embodiments of the present invention comprise methods of continuous microbial cellulose production. Many well known processes are available for fermentations where fresh nutrient is continually added to a bacterial culture while depleted nutrient containing metabolic waste products is withdrawn. When such a continuous fermentation of cellulose-producing microorganisms is conducted in contact with a structure comprising material permeable to gases and an oxygen-containing gas is circulated on one side of said material, cellulose will be continually produced on the second side of the material which contacts the culture. A continual mechanical withdrawal of cellulose so produced may be conducted and the withdrawn cellulose directly utilized or further processed.

Materials permeable to gases and suitable for use in the processes of the present invention include those comprising polyvinylchloride, cellulose, or cellulose derivatives. Further suitable gas permeable materials usable in the processes of the present invention are known in the field of blood oxygenator design as shown by Haworth (Physiological and Clinical Aspects of Oxygenator Design, (1976) eds. Davids and Engell, Elsevier/North Holland Biomedical Press, pgs. 293 to 298). These suitable gas-permeable materials include silicone rubber, silicone rubber compounded with silica particles, silicone polycarbonate copolymers, polyalkylsulphone and microporous membranes of polytetrafluoroethylene or silicone. The materials permeable to gases of the present invention may include materials comprising these described as useful in the field of blood oxygenator design.

The production of microbial cellulose by practice of the present invention may be altered by the use of agents which, alone or in combination, change the form of microbially-produced cellulose. The addition to culture media containing cellulose-producing microorganisms of various agents such as certain fluorescent brightening agents, direct cellulose dyes or cellulose derivatives alters the fibrous structure of product cellulose. Fluorescent brightening agents include those of the diaminostilbene type such as 4,4'-bis (4-anilino-6-bis 2-hydroxy-ethyl) amino-1,3,5-triazin-2-ylamino)-2,2' stilbenedisulfonic acid. Direct cellulose dyes include trypan blue, congo red, amidine red and benzo orange. Cellulose derivatives include carboxymethylcellulose, methylcellulose, and hydroxypropylcellulose (Brown et al J. Appl. Polymer Sci.: Appl. Polymer Symp. Vol. 37 pp. 33-78, 1983).

Microbial cellulose, as shaped objects, sheets, fibers or other forms produced by the practice of the present invention, may be used or processed in many different ways. Among the methods of use is the inclusion of fibrous microbial cellulose as reinforcing fiber in cementitious mixtures, as has been demonstrated with wood-pulp fiber (Coutts, et al., 1983, J. Appl. Polymer Sci.: Applied Polymer Symp., Vol. 37, pp. 829-844).

The treatment of cellulose and cellulose derivatives with liquid ammonia (L-NH$_3$) is a well known process (Herrick, idem, pp. 993-1023). Microbial cellulose produced by the process of the present invention may be likewise processed with L-NH$_3$, for example: to produce ammoniated cellulosic fibers with improved tensile strength, or reduced extensibility; to increase the structural strength of nonwoven cellulosic textiles; to increase the structural strength of nonwoven cellulosic textiles; and to form cellulosic derivatives after L-NH$_3$ cellulose activation.

A wide variety of cellulose derivatives are particularly useful to prepare liquid crystal solutions. These derivatives include various hydroxyalkyl cellulose ethers, numerous cellulose esters of carboxylic acids, cellulose sulfate, and cellulose nitrate (Gray, idem, pp. 179-192). Such derivatives may as easily be prepared from microbial cellulose produced according to the present invention and the derivatives utilized as liquid crystals.

Zugenmeier (idem, pp. 223-238) discusses the molecular conformation of cellulose esters, cellulose alkyl ethers and cellulose urethanes as disclosed by x-ray diffraction patterns.

The above cited four references, (Coutts et al.; Herrick; Gray; and Zugenmeier) all from the 1983 Journal of Applied Polymer Science are relevant to the present invention in their descriptions of cellulose derivatives and uses. Microbial cellulose produced by the processes of the present invention may he utilized for the production of woven and non-woven textiles or liquid crystals. The wide variety of cellulose derivatives available by the processes shown or discussed in the above cited references are all applicable to microbial cellulose, the particular conditions being available after only routine modifications and experimentation. The product microbial cellulose need only be subjected to the various available methods of alteration or derivatization.

The present invention offers advantages over prior related technology, which include, for example, increased rates of oxygen-dependent microbial cellulose synthesis, precise control and determination of the three dimensional shape of objects produced therewith, and the continuous production of microbial cellulose when desired.

The following examples are meant to elucidate the performance of particular embodiments of the present invention and are not meant to be limiting unless otherwise so indicated herein.

EXAMPLE 1

Sterile polyvinyl chloride gloves (Fisher Scientific 11-294-120A Disposable Vinyl Gloves) were used as shaped molds permeable to gases and subjected to the introduction of about 250 ml of Schramm & Hestrin nutrient medium (pH 5.5). Each medium-containing glove was inoculated by introducing 1.5 ml of an Acetobacter xylinum (American Type Culture Collection No. 23679) culture containing about 10$^6$ cells/ml and obtained from a four day aerobic and static culture of said microorganism in Schramm & Hestrin medium. Excess air was squeezed out of the filled gloves and the glove open ends sealed by being tied. The sealed gloves were then suspended in a one liter beaker which was covered to retain humidity. The covered beaker containing the nutrient medium and Acetobacter xylinum-containing molds was incubated at 25° C.

After three days of incubation one of the molds was removed and opened. A cellulosic object had formed on the inner surface of the mold, creating a hollow cast in the shape of a hand. The object did not stick to the mold and was readily removed intact. After six days of incubation a second mold was removed and opened. The resultant shaped object of microbial cellulose, more massive than the three day object, was readily removed and found to have completely filled finger portions while the palm region of the shaped object was yet hollow.

EXAMPLE 2

Two portions of seamless cellulose dialysis tubing having an average dry inflated diameter of about 2 cm (Fisher Scientific Cat. No. 08-667C) and referred to hereafter as 2 cm diameter dialysis tubing was immersed in distilled water and sterilized by standard autoclaving. Two portions of the dialysis tubing having lengths of about 20 cm were tied off and filled with Schramm & Hestrin nutrient medium containing about 10$^6$ Acetobacter xylinum (ATCC strain no. 23769) cells/ml. One filled tubing was suspended in an enclosed glass cylinder above a small quantity of distilled water (to maintain a humid aerobic environment) and the second filled tubing was immersed in sterile Schramm & Hestrin medium contained in an enclosed glass cylinder. Both glass cylinders and their contents were incubated for two days at about 25° C and then the filled tubing removed, opened, and their contents examined. The tubing having been suspended in the humid aerobic environment was found to be completely filled with a cylindrically shaped object of microbial cellulose. The cylindrically shaped microbial cellulose object was easily removed from the mold, had a clear jellylike center, and was whitish on the surface formerly contacting the dialysis tubing. This microbial cellulose object, about 2 cm in diameter could be twisted and stretched without breaking to a thread of less than about 1 mm diameter or could be pressed between flat surfaces into a sizeable thin sheet.

When the filled tubing which had been incubated while suspended in Schramm & Hestrin medium was opened, it was found that only a few small wisps of microbial cellulose were present. The need for an ample supply of oxygen for cellulose production was thus illustrated.

EXAMPLE 3

Another aspect of the present invention was illustrated by sterilizing a length of the 2 cm diameter dialysis tubing and then tying in a quantity of air to produce an air filled dialysis sack. The air filled sack was then immersed in Schramm & Hestrin medium held in a capped conical tube and containing about $10^6$ cells/ml of Acetobacter xylinum (American Type Culture Collection no. 23769). After incubation for a period of 3 days at about 25° C the dialysis sack was removed and found to be largely encased by a thin and clear layer of microbial cellulose.

EXAMPLE 4

A length of sterile dialysis tubing with a 2 cm diameter was filled with Schramm & Hestrin medium inoculated with about $10^6$ cells/ml of Acetobacter xylinum. The filled tubing was suspended in a capped cylinder half filled with Schramm & Hestrin medium so that the lower half of the filled tubing was immersed in the medium. After three days of incubation at about 25° C, the tubing was removed, opened and the content examined. The upper half of the tubing contained a thick cylindrical formation of microbial cellulose while the lower half had a thin membraneous formation. This observation was consistent with the apparent demand for oxygen of microbial cellulose production.

EXAMPLE 5

The potential usefulness of several materials for mold construction was evaluated. Freshly boiled distilled water was utilized to fill petri dishes which were then covered with polyvinylchloride (Fisher Scientific 11-294-12A disposable vinyl gloves); SARAN® Wrap or unstretched PARAFILM® (Amercan Can Company).

After three hours under ambient conditions the water samples in the filled covered Petri dished were measured for oxygen content by immersion therein of an oxygen electrode which was connected to an oxygen monitor. When air saturated water was calibrated to yield a reading of 100% and nitrogen saturated water, 0%; the polyvinylchloride covered sample read 86%; the SARAN® Wrap covered sample, 72%; and the PARAFILM® covered sample, 68%. The results generally indicate the material permeability to oxygen and thus suitability for use as the material permeable to gases of the present invention.

EXAMPLE 6

The formation of microbial cellulose described in EXAMPLE 3 particularly, as well as the other examples will permit the construction of apparati for continuous production of microbial cellulose. For example, a continual flow fermentation process with cellulose-producing microorganisms may be designed where a deep vat culture of Acetobacter xylinum, for example, is subjected to a continuous input of fresh liquid nutrient medium. Immersed in this deep vat liquid culture would be a multiplicity of substantially hollow columns constructed of material permeable to gases. These columns will be connected to a source of gaseous oxygen such as air for example, such that fresh oxygen will be circulated therein. The oxygen, diffusing through the permeable column walls will facilitate Acetobacter growth and cellulose production thereupon, analogous to that seen in EXAMPLE 3 herein. Harvesting the cellulose may be begun in many ways, one of which would be to initially wrap a string, for example, around the permeable column. After a cellulosic film has formed about the column, forming hydrogen bonds to the string, the string may be gradually pulled away from the column to draw a portion of the microbial cellulose deposit therewith. The cellulose deposit, having great tensile strength as described in EXAMPLE 2, for example, would follow and may then be continually withdrawn as a cellulosic fiber. The cellulosic fiber from the permeable columns may be continuously collected from the culture as individual strands from each column or as combined strands for use or further processing, for example as a textile fiber.

Changes may be made in the construction, operation and arrangement of the various segments, elements, steps and procedures described herein without departing from the scope of the invention as defined in the following claims.

**Claims**

1. A process for producing microbial cellulose characterized by:

    providing a liquid culture comprising a cellulose-producing microorganism;

    providing a structure comprising material permeable to gases but not allowing the passage of microorganisms, said structure having a first side and a second side; and

    contacting the first side with an oxygen-containing gas and the second side with the liquid culture so that microbial cellulose is produced upon the second side.

2. The process of claim 1 characterized in that the structure is a shaped mold, the culture is contained within the shaped mold and the microbial cellulose is produced as a shaped object.

3. The process of claim 1 characterized in that the structure is a shaped cast having oxygen-containing gas therein and is immersed in the culture and the microbial cellulose is produced as a coating on the cast.

4. The process of claim 1 characterized in that the process is a continuous process, the liquid culture comprises bacterial nutrient and is continually supplemented with fresh nutrient and the microbial cellulose is continually harvested.

5. The process of claim 4 defined further characterized in that the oxygen-containing gas is freshly circulated in contact with the first side.

6. The process of claim 1 characterized in that the liquid culture comprises a suitable nutrient medium.

7. The process of claim 6 characterized in that the suitable nutrient medium is Schramm-Hestrin Medium.

8. The process of claim 6 characterized in that the suitable nutrient medium comprises about 8 volume percent vinegar, 5 volume percent ethanol and 4 weight percent malt extract.

9. The process of claim 6 characterized in that the suitable nutrient medium has a pH between about 3.5 and about 5.5.

10. The process of claim 6 characterized in that the suitable nutrient medium comprises a hexose, acetic acid and yeast extract.

11. The process of claim 10 characterized in that the hexose comprises glucose.

12. The process of claim 6 characterized in that the suitable nutrient medium comprises corn steep liquor.

13. The process of claim 1 characterized in that the material comprises polyvinylchloride.

14. The process of claim 1 characterized in that the material comprises cellulose.

15. The process of claim 1 characterized in that the material comprises a cellulose derivative.

16. The process of claim 15 characterized in that the cellulose derivative is cellulose acetate or cellulose nitrate.

17. The process of claim 1 characterized in that the material comprises polyethylene.

18. The process of claim 1 characterized in that the material, comprises silicone rubber.

19. The process of claim 1 characterized in that the material comprises silicone rubber and silica particles.

20. The process of claim 1 characterized in that the material comprises silicone-polycarbonate copolymers.

21. The process of claim 1 characterized in that the material comprises polyalkylsulphone.

22. The process of claim 1 characterized in that the material comprises a microporous membrane.

23. The process of claim 1 characterized in that the microporous membrane comprises silicone.

24. The process of claim 1 characterized in that the microporous membrane comprises polytetrafluoroethylene.

25. The process of claim 1 characterized in that the microorganism is of the genus Acetobacter.

26. The process of claim 1 characterized in that the microorganism is Acetobacter xylinum.

27. The process of claim 1 characterized in that the microorganism is Acetobacter xylinum strain no. 23769 from the American Type Culture Collection.

28. The process of claim 2 characterized in that the shaped object is a contact lens.

29. The process of claim 2 defined further to include the step of impregnating the shaped object with proteins, peptides, amino acids, vitamins, other nutrients or flavoring agents individually or in combination.

**30.** The process of claim 29 characterized in that the shaped object is an artificial food or nutritional supplement.

**31.** The process of claim 1 characterized in that the microbial cellulose is a nonwoven cellulosic textile precursor.

**32.** The process of claim 2 characterized in that the culture containing shaped mold is exposed to an aerobic environment and the aerobic environment is humid.

**33.** The process of claim 1 characterized in that the contacting step is at a temperature between about 20°C and about 30°C.

**34.** The process of claim 1 characterized in that the contacting step is for a period of between about 2 days and about 21 days.

**35.** The process of claim 1 characterized in that the liquid culture comprises at least one of: a fluorescent brightening agent, a direct cellulose dye or a cellulose derivative.

**36.** The process of claim 1 defined further to include: collecting the microbial cellulose.

**37.** The process of claim 35 characterized in that the additional step is added of:

derivatizing the microbial cellulose to form a cellulose derivative.

**38.** The process of claim 36 characterized in that the cellulose derivative is an ammoniated cellulose, a cellulose ester of a carboxylic acid, a cellulose sulfate, a cellulose nitrate, a cellulose hydroxyalkyl ether or a cellulose urethane.

**39.** The process of claim 36 characterized in that the cellulose derivative is a woven or nonwoven cellulosic textile.

**40.** The process of claim 36 characterised in that the cellulose derivative is utilized as liquid crystals.

**41.** A process for producing microbial cellulose characterised by:

providing cellulose-producing microorganisms;

and

culturing said microorganisms near a material permeable to gases but not allowing the passage of microorganisms, and having an oxygen-containing gas permeate said material to supply the microorganisms with oxygen and thus facilite microbial deposition of cellulose.

**42.** A process for producing microbial cellulose characterised by:

providing a liquid culture comprising a cellulose-producing microorganism;

providing a shaped mold structure comprising material permeable to gases, the exterior of said mold structure in contact with an oxygen-containing gas, and the interior provided with said liquid culture so that microbial cellulose is produced as a shaped object on the inside of the mold..

**43.** A process for producing microbial cellulose characterized by;

providing a liquid culture comprising a cellulose-producing microorganism;

providing a shaped cast structure comprising material permeable to gases, the interior of said cast in contact with an oxygen-containing gas, and the exterior immersed in the said culture so that microbial cellulose is produced so that microbial cellulose is produced as a shaped coating on the cast.

**Revendications**

**1.** Un procédé pour produire de la cellulose microbienne caractérisé par :
   - la fourniture d'une culture liquide comprenant un microorganisme producteur de cellulose
   - la fourniture d'un matériau comprenant une structure perméable aux gaz mais ne permettant pas le passage des microorganismes, ladite structure ayant une première face et une seconde face et
   - la mise en contact de la première face avec un gaz contenant de l'oxygène et de la seconde face avec la culture liquide de telle sorte que la cellulose microbienne soit produite sur la seconde face.

**2.** Le procédé de la revendication 1, caractérisé en ce que la structure est une matrice façonnée, la culture est contenue dans la matrice façonnée et la cellulose microbienne est produite comme un objet façonné.

**3.** Le procédé de la revendication 1, caractérisé en ce que la structure est un moulage façonné ayant à l'intérieur un gaz contenant de l'oxygène et est immergée dans la culture et la cellulose microbienne est produite sous la forme d'un enrobage sur le moulage.

4. Le procédé de la revendication 1, caractérisé en ce que le procédé est un procédé en continu, la culture liquide comprend un aliment pour la bactérie et est continuellement supplée en aliment frais et la cellulose microbienne est récoltée en continu.

5. Le procédé de la revendication 4, caractérisé en outre en ce que le gaz contenant de l'oxygène est amené à circuler à l'état frais en contact avec la première face.

6. Le procédé de la revendication 1, caractérisé en ce que la culture liquide est un milieu nutritif convenable.

7. Le procédé de la revendication 6, caractérisé en ce que le milieu nutritif est le milieu de Schramm-Hestrin.

8. Le procédé de la revendication 6, caractérisé en ce que le milieu nutritif convenable comprend environ 8 pour cent en volume de vinaigre, 5 pour cent en volume d'éthanol et 4 pour cent en poids d'extrait de malt.

9. Le procédé de la revendication 6, caractérisé en ce que le milieu nutritif convenable a un pH entre environ 3,5 et environ 5,5.

10. Le procédé de la revendication 6, caractérisé en ce que le milieu nutritif convenable comprend un hexose, de l'acide acétique et de l'extrait de levure.

11. Le procédé de la revendication 10, caractérisé en ce que l'hexose comprend du glucose.

12. Le procédé de la revendication 6, caractérisé en ce que le milieu nutritif convenable contient de la liqueur de macération du maïs.

13. Le procédé de la revendication 1, caractérisé en ce que la matériau comprend du chlorure de polyvinyle.

14. Le procédé de la revendication 1, caractérisé en ce que le matériau comprend de la cellulose.

15. Le procédé de la revendication 1, caractérisé en ce que le matériau comprend un dérivé de la cellulose.

16. Le procédé de la revendication 15, caractérisé en ce que le dérivé de la cellulose est de l'acétate de cellulose ou du nitrate de cellulose.

17. Le procédé de la revendication 1, caractérisé en ce que le matériau comprend du polyéthylène.

18. Le procédé de la revendication 1, caractérisé en ce que le matériau contient un caoutchouc de silicone.

19. Le procédé de la revendication 1, caractérisé en ce que le matériau comprend un caoutchouc de silicone et des particules de silice.

20. Le procédé de la revendication 1, caractérisé en ce que le matériau comprend des copolymères silicone-polycarbonate.

21. Le procédé de la revendication 1, caractérisé en ce que le matériau comprend une polyalkylsulfone.

22. Le procédé de la revendication 1, caractérisé en ce que le matériau comprend une membrane microporeuse.

23. Le procédé de la revendication 1, caractérisé en ce que la membrane microporeuse comprend une silicone.

24. Le procédé de la revendication 1, caractérisé en ce que la membrane microporeuse comprend du polytétrafluoroéthylène.

25. Le procédé de la revendication 1, caractérisé en ce que le microorganisme est du genre Acétobacter.

26. Le procédé de la revendication 1, caractérisé en ce que le microorganisme est l'Acétobacter xylinum.

27. Le procédé de la revendication 1, caractérisé en ce que le microorganisme est l'Acétobacter xylinum souche No 23769 de l'American Type Culture Collection.

28. Le procédé de la revendication 2, caractérisé en ce que l'objet façonné est une lentille de contact.

29. Le procédé de la revendication 2, défini en outre pour inclure le stade d'imprégnation de l'objet façonné avec des protéines, des peptides, des acides aminés, des vitamines, d'autres matières nutritives ou des agents d'aromatisation individuellement ou en association.

30. Le procédé de la revendication 29, caractérisé en ce que l'objet façonné est un aliment artificiel ou un supplément nutritionnel.

31. Le procédé de la revendication 1, caractérisé en ce que la cellulose microbienne est un précurseur de textile cellulosique non-tissé.

32. Le procédé de la revendication 2, caractérisé en ce que la matrice façonnée contenant la culture est exposée à un environnement aérobe et que l'environnement aérobe est humide.

33. Le procédé de la revendication 1, caractérisé en ce que le stade de mise en contact est effectué à une température entre environ 20°C et environ 30°C.

34. Le procédé de la revendication 1, caractérisé en ce que le stade de mise en contact est effectué pendant une période comprise entre environ 2 jours et environ 21 jours.

35. Le procédé de la revendication 1, caractérisé en ce que la culture liquide comprend au moins un agent d'émission de fluorescence, un colorant direct de la cellulose ou un dérivé de cellulose.

36. Le procédé de la revendication 1, défini en outre pour inclure la recupération de la cellulose microbienne.

37. Le procédé de la revendication 35, caractérisé en ce que le stade additionnel est additionné de : la transformation de la cellulose microbienne pour former un dérivé de la cellulose.

38. Le procédé de la revendication 36, caractérisé en ce que le dérivé de cellulose est une cellulose ammoniaquée, un ester cellulosique d'un acide carboxylique, un sulfate de cellulose, un nitrate de cellulose un éther hydroxyalkylique de la cellulose ou un uréthane de cellulose.

39. Le procédé de la revendication 36, caractérisé en ce que le dérivé de cellulose est un textile cellulosique tissé ou non-tissé.

40. Le procédé de la revendication 36, caractérisé en ce que le dérivé de cellulose est utilisé comme cristaux liquides.

41. Le procédé pour produire de la cellulose microbienne caractérisé par:

- la fourniture de microorganismes producteurs de cellulose et
- la culture desdits microorganismes près d'un matériau perméable au gaz mais ne permettant pas le passage des microorganismes et ayant un gaz contenant de l'oxygène passant à travers ledit matériau pour approvisionner les microorganismes en oxygène et faciliter ainsi le dépôt microbien de cellulose.

42. Un procédé pour produire de la cellulose microbienne caractérisé par :
- la fourniture d'une culture liquide comprenant un micro-organisme producteur de cellulose,
- la fourniture d'une structure de matrice façonnée comprenant un matériau perméable aux gaz, l'extérieur de ladite structure de matrice façonnée étant en contact avec un gaz contenant de l'oxygène et l'intérieur contenant ladite culture liquide de telle sorte que la cellulose microbienne est produite sous la forme d'un objet façonné sur l'intérieur de la matrice.

43. Un procédé pour produire de la cellulose microbienne caractérisé par :
- la fourniture d'une culture liquide comprenant un microorganisme producteur de cellulose ,
- la fourniture d'une struoture de moulage façonné comprenant un matériau perméable aux gaz, l'intérieur dudit moulage étant en contact avec un gaz contenant de l'oxygène et l'extérieur étant immergé dans ladite culture de telle sorte que la cellulose microbienne soit produite sous la forme d'un enrobage sur le moulage.

**Patentansprüche**

1. Ein Verfahren zum Erzeugen von Zellulose durch Mikroben **gekennzeichnet durch:**

Vorsehen einer Flüssigkeitskultur, welche einen Zellulose produzierenden Mikroorganismus umfaßt;

Vorsehen einer Struktur, welche für Gase durchlässig ist, aber den Durchgang von Mikroorganismen nicht erlaubendes Material umfaßt, wobei die Struktur eine erste Seite und eine zweite Seite aufweist; und

Kontaktieren der ersten Seite mit einem Sauerstoff enthaltenden Gas und der zweiten Seite mit der Flüssigkeitskultur, so daß mikrobenerzeugte Zellulose auf der zweiten Seite hergestellt wird.

2. Das Verfahren nach Anspruch 1, **dadurch gekennzeichnet,** daß die Struktur eine gestaltete Form ist, daß die Kultur innerhalb der gestalteten Form enthalten ist, und daß die mikrobenerzeugte Zellulose als ein geformtes Objekt hergestellt wird.

3. Das Verfahren nach Anspruch 1, **dadurch gekennzeichnet,** daß die Struktur ein gestaltetes Formmodell ist, welches Sauerstoff enthaltendes Gas darin aufweist und in die Kultur eingetaucht wird, und daß die mikrobenerzeugte Zellulose als ein Überzug auf dem Formmodell hergestellt wird.

4. Das Verfahren nach Anspruch 1, **dadurch gekennzeichnet,** daß das Verfahren ein kontinuierlicher Prozeß ist, daß die Kultur bakteriellen Nähstoff umfaßt und kontinuierlich durch frischen Nährstoff ergänzt wird, und daß die mikrobenerzeugte Zellulose kontinuierlich geerntet wird.

5. Das Verfahren nach Anspruch 4, **dadurch gekennzeichnet,** daß das Sauerstoff enthaltende Gas frisch im Umlauf im Kontakt mit der ersten Seite gehalten wird.

6. Das Verfahren nach Anspruch 1, **dadurch gekennzeichnet,** daß die Flüssigkeitskultur ein geeignetes Nährmedium umfaßt.

7. Das Verfahren nach Anspruch 6, **dadurch gekennzeichnet,** daß das geeignete Nährmedium Schramm-Hestrin-Medium ist.

8. Das Verfahren nach Anspruch 6, **dadurch gekennzeihnet,** daß das geeignete Nährmedium etwa 8 Volumenprozent Essig, 5 Volumenprozent Äthanol und 4 Gewichtsprozent Malzextrakt enthält.

9. Das Verfahren nach Anspruch 6, **dadurch gekennzeichnet,** daß das geeingete Nährmedium einen pH-Wert zwischen etwa 3,5 und etwa 5,5 aufweist.

10. Das Verfahren nach Anspruch 6, **dadurch gekennzeichnet,** daß das geeingete Nährmedium eine Hexose, Essigsäure und Hefeextrakt umfaßt.

11. Das Verfahren nach Anspruch 10, **dadurch gekennzeichnet,** daß die Hexose Glukose umfaßt.

12. Das Verfahren nach Anspruch 6, **dadurch gekennzeichnet,** daß das geeingete Nährmedium Flüssigkeit mit eingeweichtem Getreide umfaßt.

13. Das Verfahren nach Anspruch 1, **dadurch gekennzeichnet,** daß das Material Polyvenylchlorid umfaßt.

14. Das Verfahren nach Anspruch 1, **dadurch gekennzeichnet,** daß das Material Zellulose umfaßt.

15. Das Verfahren nach Anspruch 1, **dadurch gekennzeichnet,** daß das Material Zellulosederivat umfaßt.

16. Das Verfahren nach Anspruch 15, **dadurch gekennzeichnet,** daß das Zellulosederivat Zelluloseacetat oder Zellulosenitrat ist.

17. Das Verfahren nach Anspruch 1, **dadurch gekennzeichnet,** daß das Material Polyethylen umfaßt.

18. Das Verfahren nach Anspruch 1, **dadurch gekennzeichnet,** daß das Material Silikongummi umfaßt.

19. Das Verfahren nach Anspruch 1, **dadurch gekennzeichnet,** daß das Material Silikongummi und Kieselerdepartikel umfaßt.

20. Das Verfahren nach Anspruch 1, **dadurch gekennzeichnet,** daß das Material Silikon-Polykarbonatkopolymere umfaßt.

21. Das Verfahren nach Anspruch 1, **dadurch gekennzeichnet,** daß das Material Polyalkylsulfon umfaßt.

22. Das Verfahren nach Anspruch 1, **dadurch gekennzeichnet,** daß das Material eine mikroporöse Membran aufweist.

23. Das Verfahren nach Anspruch 1, **dadurch gekennzeichnet,** daß die mikroporöse Membran Silikon umfaßt.

24. Das Verfahren nach Anspruch 1, **dadurch gekennzeichnet,** daß die mikroporöse Membran Polytetrafluorethylen umfaßt.

25. Das Verfahren nach Anspruch 1, **dadurch gekennzeichnet,** daß der Mikroorganismus von der Gattung Acetobacter ist.

26. Das Verfahren nach Anspruch 1, **dadurch gekennzeichnet,** daß der Mikroorganismus Acetobacterxylinum ist.

27. Das Verfahren nach Anspruch 1, **dadurch gekennzeichnet,** daß der Mikroorganismus Acetobacterxylinium Züchtungsstamm-Nummer 23769 aus der Kulturensammlung vom amerikanischen Typ ist.

28. Das Verfahren nach Anspruch 2, **dadurch gekennzeichnet,** daß das gestaltete Objekt eine Kontaktlinse ist.

29. Das Verfahren nach Anspruch 2, ferner dadurch definiert, daß es den Schritt der Imprägnierung des gestalteten Objekts mit Proteinen, Peptiden, Aminosäuren, Vitaminen, anderen Nährmitteln oder Aromastoffen einzeln oder in Kombination enthält.

30. Das Verfahren nach Anspruch 29, **dadurch gekennzeichnet,** daß das gestaltete Objekt ein künstliches Nahrungsmittel oder eine Ernährungsergänzung ist.

31. Das Verfahren nach Anspruch 1, **dadurch gekennzeichnet,** daß die mikrobenerzeugte Zellulose ein nicht gewebtes auf Zellulose basierendes textiles Zwischenprodukt ist.

32. Das Verfahren nach Anspruch 2, **dadurch gekennzeichnet,** daß die die Kultur enthaltende gestaltete Form einer aerobischen Umgebung ausgesetzt ist und die aerobische Umgebung feucht ist.

33. Das Verfahren nach Anspruch 1, **dadurch gekennzeichnet,** daß der Kontaktierungsschritt bei einer Temperatur zwischen etwa 20° C und etwa 30° C erfolgt.

34. Das Verfahren nach Anspruch 1, **dadurch gekennzeichnet,** daß der Kontaktierungsschritt für einen Zeitraum zwischen etwa 2 Tagen und etwa 21 Tagen erfolgt.

35. Das Verfahren nach Anspruch 1, **dadurch gekennzeichnet,** daß die Flüssigkeitskultur wenigstens eine der folgenden Komponenten umfaßt: ein fluoreszierendes Aufhellmittel, ein Zellulosedirektfarbstoff oder ein Zellulosederivat.

36. Das Verfahren nach Anspruch 1, **dadurch gekennzeichnet,** daß es weiterhin das Sammeln der mikrobenerzeugten Zellulose enthält.

37. Das Verfahren nach Anspruch 35, **dadurch gekennzeichnet,** daß dem zusätzlichen Schritt hinzugefügt wird: Verändern der mikrobenerzeugten Zellulose, um ein Zellulosederivat zu bilden.

38. Das Verfahren nach Anspruch 36, **dadurch gekennzeichnet,** daß das Zellulosederivat eine ammonierte Zellulose, ein Zelluloseester einer Karbonsäure, ein Zellulosesulfat, ein Zellulosenitrat, ein Zellulosehydroxyalkylester oder ein Zelluloseurethan ist.

39. Das Verfahren nach Anspruch 36, **dadurch gekennzeichnet,** daß das Zellulosederivat ein gewebtes oder nicht gewebtes Zellulosetextilmaterial ist.

40. Das Verfahren nach Anspruch 36, **dadurch gekennzeichnet,** daß das Zellulosederivat als Flüssigkristalle verwendet wird.

41. Ein Verfahren zum Herstellen von mikrobenerzeugter Zellulose, **gekennzeichnet durch:**

Bereitstellen Zellulose produzierender Mikroorganismen; und

Kultivieren der Mikroorganismen nahe einem Material, das für Gase durchlässig ist, aber nicht den Durchgang von Mikroorganismen erlaubt, und Vorsehen eines das Material durchdringenden Sauerstoff enthaltenden Gases, um die Mikroorganismen mit Sauerstoff zu versorgen und so eine mikrobenerzeugte Ablagerung von Zellulose zu fördern.

42. Ein Verfahren zum Herstellen mikrobenerzeugter Zellulose, **gekennzeichnet durch:**

Bereitstellen einer Flüssigkeitskultur, die einen Zellulose produzierenden Mikroorganismus umfaßt;

Vorsehen einer gestalteten Formstruktur, welche für Gase durchlässiges Material umfaßt, wobei das Äußere der Formstruktur in Kontakt mit einem Sauerstoff enthaltenden Gas ist, und das Innere mit der Flüssigkeitskultur versehen ist, so daß mikrobenerzeugte Zellulose als ein geformtes Objekt auf der Innenseite der Form erzeugt wird.

43. Ein Verfahren zum Herstellen mikrobenerzeugter Zellulose, **gekennzeichnet durch:**

Vorsehen einer Flüssigkeitskultur, die einen Zellulose produzierenden Mikroorganismus umfaßt;

Vorsehen einer gestalteten Formmodellstruktur, die ein für Gase durchlässiges Material umfaßt, wobei das Innere der Struktur in Kontakt mit einem Sauerstoff enthaltenden Gas ist, und wobei das Äußere in die Kultur eingetaucht wird, so daß mikrobenerzeugte Zellulose als ein geformter Überzug auf der Struktur erzeugt wird.